Europäisches Patentamt

European Patent Office ·

Office européen des brevets

(19)

(11) Publication number: **0 057 561**
**B1**

(12) **EUROPEAN PATENT SPECIFICATION**

(45) Date of publication of patent specification: **16.04.86**

(21) Application number: **82300394.2**

(22) Date of filing: **26.01.82**

(51) Int. Cl.⁴: **A 61 N 1/36**

(54) **Muscle stimulating apparatus.**

(30) Priority: **29.01.81 GB 8102740**

(43) Date of publication of application:
**11.08.82 Bulletin 82/32**

(45) Publication of the grant of the patent:
**16.04.86 Bulletin 86/16**

(84) Designated Contracting States:
**AT BE CH DE FR GB IT LI LU NL SE**

(56) References cited:
**EP-A-0 000 477**
**DE-A-2 437 346**
**FR-A-2 433 950**
**US-A-2 568 934**
**US-A-3 851 651**
**US-A-4 177 819**

**MEDICAL & BIOLOGICAL ENGINEERING AND COMPUTING, vol. 17, no. 3, May 1979, pages 421-424, Stevenage (GB); J.S. PETROFSKY: "Digital-analogue hybrid 3-channel sequential stimulator"**

(73) Proprietor: **BIO MEDICAL RESEARCH LIMITED**
**Bay 82 Shannon Industrial Estate**
**Shannon Airport County Clare (IE)**

(72) Inventor: **Matthews, Clive Royston**
**Rathluby Kilmurry**
**Ennis Clare (IE)**
Inventor: **Quinlan, Michael Martin**
**Mount Laurence**
**Caherconlish Limerick (IE)**
Inventor: **O'Connor, Maurice Gerard**
**22, Shannon Grove Shannon**
**Banks Corbally Limerick (IE)**

(74) Representative: **Ayers, Martyn Lewis Stanley et al**
**J.A. KEMP & CO. 14 South Square Gray's Inn**
**London, WC1R 5EU (GB)**

EP 0 057 561 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a muscle stimulating apparatus.

Muscle stimulating apparatuses are well-known which comprise a plurality of electrodes which are applied to the skin and a pulse source which supplies to them a stream of pulses of suitable frequency, amplitude and waveform to stimulate muscles below the skin for the purposes of toning or exercising them. One such apparatus is the Slendertone (Registered Trade Mark).

Multiple-channel muscle stimulators are known which have a number of pairs of electrodes to which a stimulating pulse train is applied. Where a number of pairs of electrodes is used the problem arises that by mis-use or incorrect connection of the electrodes to the pulse source, it is possible for undesirable effects to occur.

US 3851651 discloses a muscle stimulator according to the precharacterizing part of claim 1. The multiple outputs are intended to enable different muscle groups to be stimulated at the same time from a common pulse source. The disclosure is not concerned with electrode-pair interactions or the stimulation of muscles of the same muscle group.

According to the present invention we provide muscle stimulating apparatus for stimulation of a person's muscles comprising: a source of electrical signals which, in use, is located exteriorly of the person's body; a plurality of electrodes adapted to be applied adjacent respective locations on the epidermis of the person to stimulate underlying muscles; the source of electrical signals comprising a pulse generator for producing a train of muscle stimulating pulses, means for producing amplified bursts of the pulses and means for multiplexing the pulses into a number of separate trains of pulses for application to respective electrodes, characterised in that the means for producing amplified bursts includes means for modulating the envelopes of the bursts and in that the multiplexing means is arranged to multiplex the envelope modulated bursts such that successive pulses of each burst are applied across the electrodes a pair at a time in a predetermined time sequence.

It may be noted that the article "Digital-analogue hybrid 3-channel sequential stimulator" by J. S. Petrofsky, Medical & Biological Engineering & Computing, May 1979, pages 421—424 discloses a multiplexed muscle stimulator with pulse envelope modulation. However, the effect of the arrangement disclosed is that the three separate trains of pulses cooperate so as effectively to stimulate the muscle at a frequency higher than that of the pulse train.

One suitable form for the applied signals is bursts followed by spaces of lengths approximately equal to the bursts. Preferably, the leading edge of the envelope of each burst is rounded by the modulating means so that the burst surges to its maximum value.

One way of producing such a waveform with the present apparatus is to have a pulse generator which generates the pulses at a desired frequency (e.g. 100/Mn) and to amplitude modulate it with the output of a much lower frequency square waveform from an envelope timing generator and having a suitably rounded leading edge. The resulting modulated pulse train is applied via an amplifier/buffer to the primary of an output transformer. The output of the higher frequency pulse generator is also connected to a pulse counter and "One of N" decoder, where N is the desired number of outputs of the apparatus. Associated with the secondary of the output transformer is multiplexing circuitry suitable for deriving N separate pulse streams, one for each set of electrodes. These pulse streams are derived using N electronic switches, preferably opto-isolators, each controlled by individual signals of the outputs of the "One of N" decoder and are delivered to respective output terminals for connection to the body electrodes. In the circuitry associated with the transformer secondary, each set of electrodes may share a common return line and this can include a current limiting device such as a controlled current source to ensure that the maximum current is limited to a desirable and safe level.

An apparatus as described above is described in more detail below with reference to the accompanying drawings. The illustrated apparatus has a number of advantages. Because of the time division multiplex method of production of the pulse streams and the inclusion of the current limiting device, the pulses applied to each set of electrodes have a known and limited maximum energy content. The apparatus is incapable of providing peak voltages and currents beyond those supplied by the transformer and current limiting device, regardless of any accidental or deliberate cross connecting of any output electrode groups. Electrical interaction between electrodes of different sets when placed on the skin for the purpose of stimulation cannot occur because the pulse signals to each arrive at the electrodes at different times, therefore the effects of having signals present on all outputs simultaneously, which is mentioned above has caused interaction problems in the past, is eliminated.

The above and other features of the invention will be further described by way of non-limiting example with reference to the accompanying drawings, in which:—

FIGURE 1 is a functional block diagram of one embodiment of the invention;

FIGURE 2 shows the waveforms occurring at various points in the circuitry of Figure 1; and

FIGURE 3 shows one particular form of the circuitry of Figure 1.

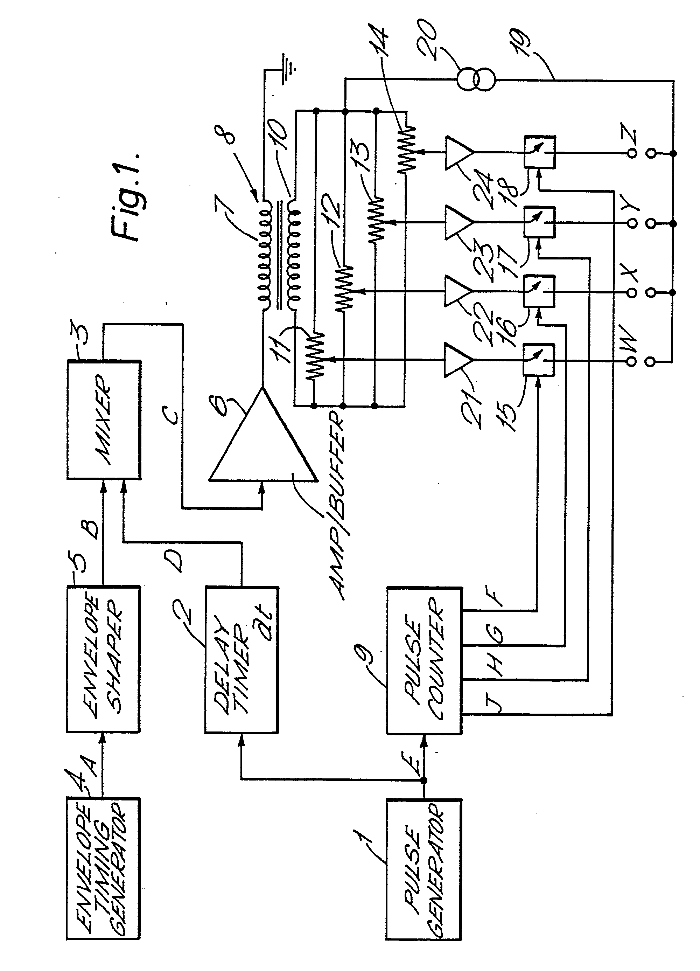

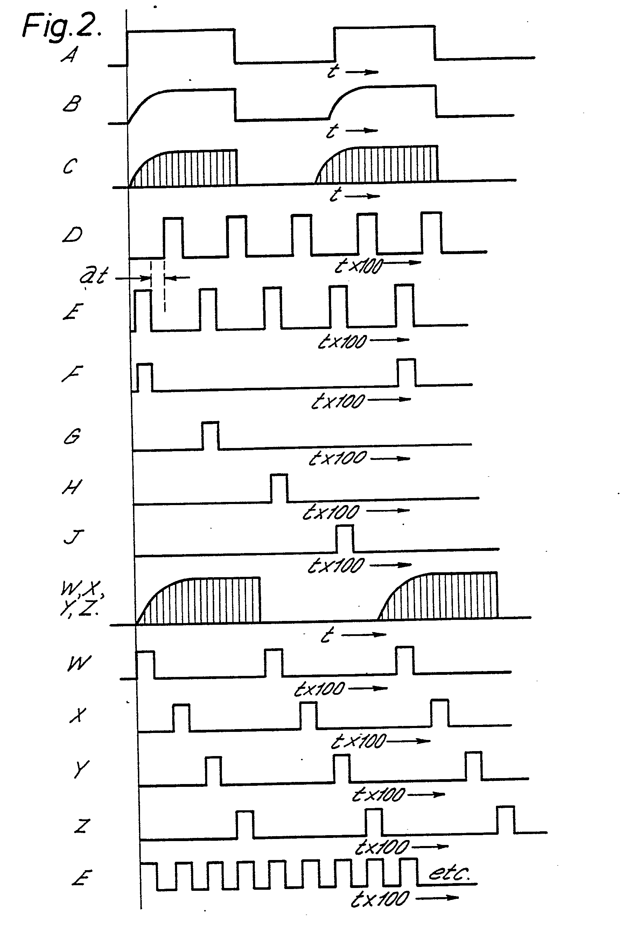

The general construction and operation of the illustrated apparatus should be readily apparent from Figures 1 and 2.

Briefly, the apparatus comprises a pulse generator 1 whose output is delivered by a delay circuit which provides a short delay to equalise delay times throughout the circuitry to a mixer 3 where the pulse stream D is amplitude modulated with a much lower frequency pulse waveform B. This waveform B has an approximately 50 percent duty cycle and rounded positive-going edges and is derived from a lower frequency pulse generator 4 by an envelope shaper 5. The output from mixer 3 is applied to the input of an amplifier and buffer 6 which drives the primary 7 of an output transformer 8.

The output of pulse generator 1 is also applied to the input of a pulse counter 9 whose outputs F to J each go high in succession to produce 4 streams of pulses as indicated at F to J in Figure 2 which go high in non-overlapping relation to one another and in synchronism with respective ones of 4 successive pulses of the pulse train E. The pulse counter may, for example, comprise a 4-bit recycling counter whose outputs are connected to a "One-of-4" decoder.

Connected in parallel across the secondary 10 of transformer 8 are four potentiometers 11 to 14 associated with respective output terminals W—Z to which, in use, may be connected one electrode of each of 4 sets of electrodes which may be applied to the body of the user using suitable body pads. The wipers of the potentiometers 11 to 14 are connected to the associated terminals W—Z via respective electronically operated switches 15 to 18 which are controlled by the individual ones of the outputs F to J of pulse counter 9. These electronic switches may be of the opto-isolator type e.g. having a light emitting diode driven by the associated output of pulse counter 9 and a phototransistor constituting the controlled-conduction path. This, of course, serves to ensure that the circuitry connected to the body electrodes "floats" for isolation purposes.

The outputs W—Z may share a common return line 19 to the secondary 10 of the transformer and this may include a current limiting device 20 such as a constant current source to keep the pulsed currents at a desired value.

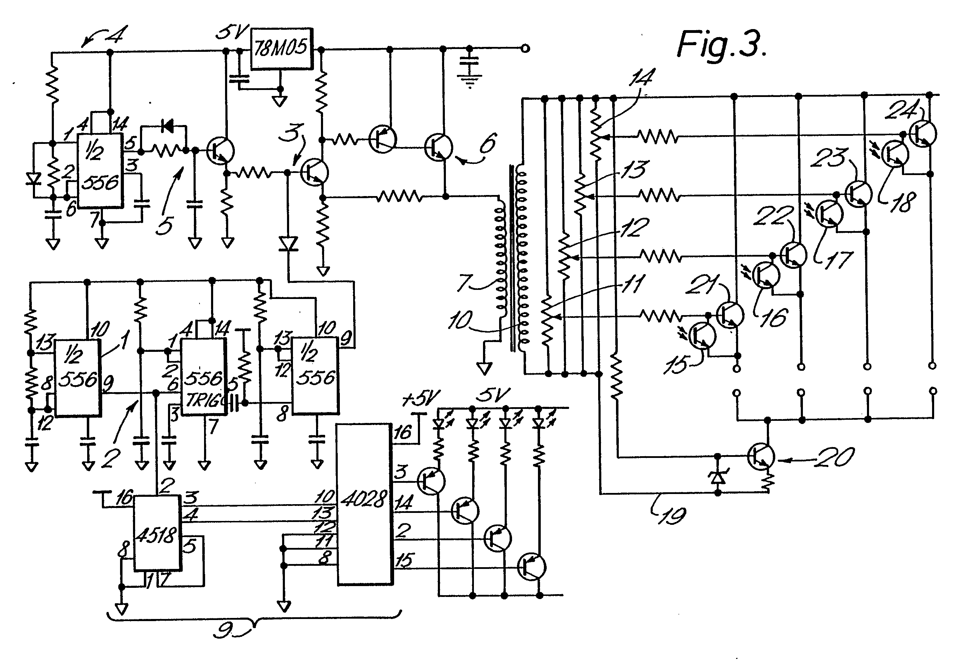

In Figure 1 each potentiometer 11—14 is shown connected to its associated output W—Z via an amplifier 21—24 and switch 15—18. This arrangement of the amplifiers and switches is intended to be schematic in the corresponding part of Figure 3; the controlled element of each opto-isolator is a photo transistor 15—18 which when turned on shunts the collector to emitter circuit of an associated transistor 21—24 which corresponds to the amplifier in Figure 2. These transistors 21—24 are suitably rated to handle the output voltage from transformer 8.

Figure 3 shows one possible implementation of the circuitry of Figure 1 using "556" timers, CD4000 series logic circuitry and opto-isolators.

By way of example, suitable signal characteristics are as follows:—

Pulse duration (as in D.E.W,X,Y,Z) from 20 to 200 microseconds;
Pulse interval (as in W.X,Y,Z) 12 milliseconds;
Pulse interval (as in E,D) 3 milliseconds for 4 output circuit version of the stimulating device or
Pulse interval (as E,D) 1.5 milliseconds for 8 output circuit version of the stimulating device.
Delay time (E&D) 200 microseconds;
Pulse train duration (as in A,B,C) 1 second;
Pulse train interval (as in A,B,C) 1.5 second;
Maximum output voltage 110V into a 1 kilohm load;
Maximum output current 120 milliamps.

Other values for the above parameters are, of course, possible: although in the illustrated embodiment the pulses applied to the body electrodes are unipolar, the circuitry could be so arranged that the pulses are bipolar.

## Claims

1. Muscle stimulating apparatus for subcutaneous stimulation of a person's muscles comprising: a source of electrical signals which, in use, is located exteriorly of the person's body; a plurality of electrodes adapted to be applied adjacent respective locations on the epidermis of the person to stimulate underlying muscles; the source of electrical signals comprising a pulse generator (1) for producing a train of muscle stimulating pulses, means (3, 6) for producing amplified bursts of the pulses and means (9, 11—18, 21—24) for multiplexing the pulses into a number of separate trains of pulses for application to respective electrodes, characterised in that the means for producing amplified bursts includes means (3, 4, 5) for modulating the envelopes of the bursts and in that the multiplexing means is arranged to multiplex the envelope modulated bursts such that successive pulses of each burst are applied across the electrode a pair at a time in a predetermined time sequence.

2. Apparatus according to claim 1 wherein the multiplexing means comprises a plurality of switches (15—18) or corresponding circuits in one-to-one relation with the electrode pairs outputs, and means (9) for sequentially actuating said switches or circuits in response to the occurrence of said electrical signals thereby causing said electrical signals to energize each electrode pair as the switch corresponding to its respective output is actuated.

3. Apparatus according to claim 2 wherein each of said switches (15—18) comprises a light sensitive switching element connected in circuit between said source of electrical signals and a respective one of said electrode pairs, and a light source which is operatively connected to said source of electrical signals and energized in response to said electrical signals.

4. Apparatus according to any one of the preceding claims wherein the electrodes are arranged in pairs, there being a "line" electrode and a "return" electrode in each pair and the return electrodes of the pair being electrically connected together.

5. Apparatus according to any one of the preceding claims and including current limiting means (20) for limiting the current flowing through the user's body to below a predetermined magnitude.

6. Apparatus according to claims 4 and 5 wherein there is a common return circuit path (19) connected between said source of electrical signals and the return electrodes and the current limiting means (20) is connected in said common return circuit path for maintaining the current between the electrodes of each of said electrode pairs below a predetermined magnitude.

7. Apparatus according to any one of the preceding claims wherein said source of electrical signals comprises a pulse counter (9) having an input connected to said pulse generator (1) and a plurality of outputs (F,G,H,J), and a plurality of switches (15—18) each of which is operatively connected between a respective one of the outputs of said pulse counter and one of said electrode pairs, each of said switches being actuated in response to the signal at its respective pulse counter output.

**Revendications**

1. Appareil de stimulation musculaire pour la stimulation sous-cutanée des muscles d'une personne, comportant: une source de signaux électriques qui, en service, est située à l'extérieur du corps de la personne; plusieurs électrodes adaptées pour être appliquées adjacentes à des emplacements respectifs sur l'épiderme de la personne pour stimuler les musles sous-jacents; la source de signaux électriques comportant un générateur d'impulsions (1) pour produire un train d'impulsions de stimulation musculaire, des moyens (3, 6) pour produire des rafales amplifiées d'impulsions et des moyens (9, 11—18, 21—24) pour multiplexer les impulsions en un certain nombre de trains séparés d'impulsions pour être appliquées aux électrodes respectives, caractérisé en ce que les moyens pour produire les rafales amplifiées comportent des moyens (3, 4, 5) pour moduler les enveloppes des rafales et en ce que les moyens de multiplexage sont disposés pour multiplexer les rafales à enveloppe modulée de façon que les impulsions successives de chaque rafale soient appliquées aux électrodes, une paire à la fois, selon une séquence chronologique déterminée.

2. Appareil selon la revendication 1, dans lequel les moyens de multiplexage comportent une multiplicité d'interrupteurs (15—18) ou de circuits correspondants en relation univoque avec les sorties des paires d'électrodes, et des moyens (9) pour actionner séquentiellement ces interrupteurs ou ces circuits en réponse à l'apparition des signaux électriques, d'où il résulte que ces signaux électriques excitent chaque paire d'électrodes lorsque l'interrupteur correspondant à sa sortie respective est actionné.

3. Appareil selon la revendication 2, dans lequel chacun des interrupteurs (15—18) comporte un élément interrupteur photosensible monté en circuit entre la source de signaux électriques et l'une respective de ces paires d'électrodes, et une source lumineuse qui est raccordée fonctionellement à cette source de signaux électriques et excitée en réponse à ces signaux électriques.

4. Appareil selon l'une quelconque des revendications précédentes, dans lequel les électrodes sont disposées par paires, chaque paire comprenant une électrode "de ligne" et une électrode "de retour" et les électrodes de retour des paires étant électriquement raccordées ensemble.

5. Appareil selon l'une quelconque des revendications précédentes, comprenant des moyens limiteurs de courant (20) pour limiter le courant circulant à travers le corps de l'utilisateur à une valeur inférieure à une valeur prédéterminée.

6. Appareil selon les revendications 4 et 5, dans lequel il y a un trajet de circuit de retour commun (19) monté entre cette source de signaux électriques et les électrodes de retour, et les moyens limitateurs de courant (20) sont montés dans le trajet de circuit de retour commun pour maintenir le courant entre les électrodes de chaque paire d'électrodes en dessous d'une valeur prédéterminée.

7. Appareil selon l'une quelconque des revendications précédentes, dans lequel la source de signaux électriques comporte un compteur d'impulsions (9) ayant une entrée reliée au générateur d'impulsions (1) et une multiplicité de sorties (F, G, H, J) et une multiplicité d'interrupteurs (15—18) dont chacun est fonctionnellement monté entre l'une respective des sorties du compteur d'impulsions et l'une des paires d'électrodes, chacun des interrupteurs étant actionné en réponse au signal au niveau de sa sortie respective du compteur d'impulsions.

**Patentansprüche**

1. Muskelstimulationsgerät für die subkutane Stimulation der Muskeln einer Person mit einer Quelle für elektrische Signale, die im Gebrauch außerhalb der Körpers der Person angeordnet ist, und mit einer Mehrzahl von Elektroden, die nahe bestimmten Stellen auf der Epidermis der Person zur Stimulierung darunter liegender Muskeln angelegt werden können, wobei die Quelle der elektrischen Signale einen eine Reihe von muskelstimulierenden Impulsen erzeugenden Impulsgenerator (1), Einrichtungen (3, 6) zur Erzeugung verstärker Stöße der Impulse und Einrichtungen (9, 11—18, 21—24) zum Multiplexen der Impulse in eine Anzahl von getrennten Impulsreihen zum Anlegen an jeweilige Elektroden umfaßt, dadurch gekennzeichnet, daß die Einrichtungen zur Erzeugung der verstärkten Stöße die Hüllen der Stöße modulierende Bauelemente (3, 4, 5) umfassen und daß die Multiplexereinrichtung zum Multiplexen der hüllenmodulierten Stöße derart angeordnet ist, daß aufeinanderfolgende Impulse jedes Stoßes in einer vorbestimmten Zeitfolge parallel an ein Elektrodenpaar auf einmal angelegt werden.

2. Gerät nach Anspruch 1, dadurch gekennzeichnet, daß die Multiplexereinrichtung eine Mehrzahl von Schaltern (15—18) oder entsprechende Schaltungen in einer 1:1-Beziehung mit den Elektrodenpaarausgängen und ein Bauelement (9) für die aufeinanderfolgende Betätigung dieser Schalter oder Schaltungen im Ansprechen auf das Auftreten der elektrischen Signale umfaßt, so daß die elektrischen Signale jedes Elektrodenpaar aur Erregung bringen, wenn der ihrem jeweiligen Ausgang entsprechende Schalter betätigt wird.

3. Gerät nach Anspruch 2, dadurch gekennzeichnet, daß jeder der Schalter (15—18) ein lichtempfindliches, in den Kreis zwischen der Quelle der elektrischen Signale und einem der jeweiligen Elektrodenpaare einbezogenes Schaltelement sowie eine betrieblich mit der Quelle der elektrischen Signale verbundene Lichtquelle, die im Ansprechen auf die elektrischen Signale erregt wird, umfaßt.

4. Gerät nach einem der vorherigen Ansprüche, dadurch gekennzeichnet, daß die Elektroden paarweise angeordnet sind und in jedem Paar eine "Leitungs"-Elektrode sowie eine "Rückführ"-Elektrode vorhanden ist, wobei die Rückführelektroden des Paars elektrisch miteinander verbunden werden.

5. Gerät nach einem der vorherigen Ansprüche, gekennzeichnet, durch eine Strombegrenzungseinrichtung (20), die den durch den Körper der Person fließenden Strom unter eine vorbestimmte Größe beschränkt.

6. Gerät nach Anspruch 4 und 5, dadurch gekennzeichnet, daß eine zwischen die Quelle der elektrischen Signale sowie die Rückführ-Elektroden geschalteter gemeinsamer Rückführ-Strompfad (19) vorhanden ist und daß die Strombegrenzungseinrichtung (20) in den gemeinsamen Rückführ-Strompfad geschaltet ist, um den Strom zwischen den Elektroden eines jeden der Elektrodenpaare unter einer vorbestimmten Größe zu halten.

7. Gerät nach einem der vorhergehenden Ansprüche, dadurch gekennzeichnet, daß die Quelle der elektrischen Signale einen Impulszähler (9) mit einem mit dem Impulsgenerator (1) verbundenen Eingang sowie mit einer Mehrzahl von Ausgängen (F, G, H, I) und eine Mehrzahl von Schaltern (15—18) umfaßt, von denen jeder betrieblich zwischen jeweils einen der Ausgänge des Impulszählers sowie eines der Elektrodenpaare geschaltet ist und jeder der Schalter im Ansprechen auf das Signal an seinem jeweiligen Impulszählerausgang betätigt wird.

Fig.1.

0 057 561

Fig.2.

0 057 561

2

Fig.3.